# EUROPEAN PATENT SPECIFICATION

(11) **EP 2 907 460 B1**
(45) Date of publication and mention of the grant of the patent: **18.09.2019**
(21) Application number: 15153886.5
(22) Date of filing: 05.02.2015
(51) Int. Cl.: A61B 17/32

(54) **Surgical shaver assembly**
Chirurgische Resektionsanordnung
Ensemble de résection chirurgical

(30) Priority: 12.02.2014 US 201461938803 P; 24.10.2014 US 201414522620
(43) Date of publication of application: 19.08.2015
(73) Proprietor: Arthrex, Inc., Naples, FL 34108-1945 (US)
(72) Inventor: Bickenbach, Christine, Naples, FL 34119 (US)
(74) Representative: Rankin, Douglas

(56) References cited:
- US-A- 4 167 943
- US-A- 4 850 354
- US-A- 5 437 630
- US-A- 5 474 532
- US-A- 5 741 287
- US-A- 5 766 199
- US-A1- 2007 282 361

## Description

### BACKGROUND

This disclosure relates to a surgical device, and more particularly to a surgical shaver assembly for severing tissue at an internal surgical site.

Arthroscopic and endoscopic procedures are commonly performed to diagnose and treat problems in joints. For example, ligament reconstruction, bone resurfacing and joint replacement may all be performed using arthroscopic reconstruction procedures that typically require a surgeon to work through a series of relatively small portals. It may become necessary during such procedures to remove tissue or debris that surrounds the joint space under repair. Although surgical shavers are known, additional advancements in this field of technology are desirable. US 5 741 287 A discloses a surgical shaver assembly, comprising an outer tube, an inner tube having a longitudinal axis, a proximal end and a distal end rotatably received within a hollow passage of said outer tube, said inner tube including a distal portion and a window that extends through said distal portion wherein the inner and outer tube have tapered distal sections.

### SUMMARY

The invention is defined in claim 1. Preferred embodiments of the invention are defined in the dependent claims. A surgical shaver assembly according to an exemplary aspect of the present disclosure includes, among other things, an outer tube and an inner tube rotatably received within a hollow passage of the outer tube. The inner tube includes a distal portion and a window that extends through the distal portion.

In a further non-limiting embodiment of the foregoing assembly, the inner tube includes a tubular body that includes the distal portion, the distal portion tapering in direction that extends from the tubular body toward a distal-most end of the inner tube.

In a further non-limiting embodiment of either of the foregoing assemblies, the inner tube includes a top surface including a top opening and a bottom surface including a bottom opening, the window extending from the top opening to the bottom opening.

In a further non-limiting embodiment of any of the foregoing assemblies, cutting edges circumscribe the periphery of both the top opening and the bottom opening.

In a further non-limiting embodiment of any of the foregoing assemblies, the outer tube includes a top surface that includes a top opening and a bottom surface that is closed, the top opening establishing a window of the outer tube.

In a further non-limiting embodiment of any of the foregoing assemblies, the inner tube is configured to rotate within the outer tube to sever tissue that extends through the window of the outer tube and the window of the inner tube when the windows become aligned.

In a further non-limiting embodiment of any of the foregoing assemblies, the inner tube includes a bottom opening and the outer tube includes a bottom surface. A gap extends between the bottom opening and the bottom surface.

In a further non-limiting embodiment of any of the foregoing assemblies, the inner tube is self-centering relative to the outer tube to establish a tight gap between therebetween. The tight gap induces a scissor effect when the inner tube rotates within the outer tube to sever tissue.

In a further non-limiting embodiment of any of the foregoing assemblies, a top surface of the outer tube tapers at a first taper angle toward a distal-most end of the outer tube to establish a window of the outer tube.

In a further non-limiting embodiment of any of the foregoing assemblies, a bottom surface of the outer tube includes an arc that rotates around a longitudinal axis of the outer tube to configure a distal portion of the outer tube in a "torpedo" shape.

In a further non-limiting embodiment of any of the foregoing assemblies, the outer tube includes a window that extends into a portion of a tubular body of the outer tube that is proximal to a distal portion of the outer tube.

In a further non-limiting embodiment of any of the foregoing assemblies, the window of the inner tube includes a first width at a proximal side and a second width at a distal side. The first width is larger than the second width.

In a further non-limiting embodiment of any of the foregoing assemblies, the inner tube includes a tubular body that establishes a hollow passage, and a distal opening of the hollow passage opens into the window of the inner tube.

In a further non-limiting embodiment of any of the foregoing assemblies, the distal opening includes a V-shaped cross-section.

In a further non-limiting embodiment of any of the foregoing assemblies, the distal portion of the inner tube includes a top surface and a bottom surface that include proximal sections that converge toward one another, intermediate sections that diverge away from one another, and distal sections that converge toward one another.

A surgical method according to another exemplary aspect of the present disclosure includes among other things, positioning a surgical shaver assembly within a joint space. The surgical shaver assembly includes an inner tube rotatably received within an outer tube. The inner tube includes a distal portion and a window that extends through the distal portion. The method further includes rotating the inner tube relative to the outer tube of the surgical shaver assembly to sever tissue of the joint space.

In a further non-limiting embodiment of the foregoing surgical method, the rotating step includes severing tissue that extends into the window of the inner tube.

In a further non-limiting embodiment of either of the foregoing surgical methods, the rotating step includes moving the inner tube along a cutting edge path such that the window of the inner tube and another window of the outer tube align to allow the tissue to protrude into the windows and subsequently be severed as the inner tube continues along the cutting edge path.

In a further non-limiting embodiment of any of the foregoing surgical methods, the inner tube is self-centering relative to the outer tube to establish a tight gap between the inner tube and the outer tube. The tight gap induces a scissor effect between the inner tube and the outer tube during the rotating step.

In a further non-limiting embodiment of any of the foregoing surgical methods, the inner tube includes a top surface including a top opening and a bottom surface including a bottom opening, the window extending from the top opening to the bottom opening.

The embodiments, examples and alternatives of the preceding paragraphs, the claims, or the following description and drawings, including any of their various aspects or respective individual features, may be taken independently or in any combination. Features described in connection with one embodiment are applicable to all embodiments, unless such features are incompatible.

The various features and advantages of this disclosure will become apparent to those skilled in the art from the following detailed description. The drawings that accompany the detailed description can be briefly described as follows.

### BRIEF DESCRIPTION OF THE DRAWINGS

Figure 1 illustrates a joint space being prepared for a surgical procedure using a surgical shaver assembly.
Figure 2 illustrates a surgical shaver assembly.
Figure 3 schematically illustrates rotation of an inner tube relative to an outer tube of a surgical shaver assembly.
Figure 4 illustrates an exploded view of portions of a surgical shaver assembly.
Figure 5 illustrates a cross-sectional view of a surgical shaver assembly.
Figures 6A, 6B and 6C illustrate multiple views of an outer tube of a surgical shaver assembly.
Figures 7A, 7B, 7C and 7D illustrate multiple views of an inner tube of a surgical shaver assembly.
Figure 8 illustrates a cross-sectional view of a surgical shaver assembly.

### DETAILED DESCRIPTION

This disclosure relates to a surgical shaver assembly. The surgical shaver assembly can be used to prepare a joint space for a surgery. For example, the surgical shaver assembly could be used to clean and prepare the joint space for an endoscopic or arthroscopic procedure. The exemplary surgical shaver assembly includes an inner tube that is rotatable within an outer tube along a cutting edge path to sever tissue that protrudes into aligned windows of the surgical shaver assembly. Tip portions of both the inner tube and the outer tube may include a "torpedo" shape that is adapted to easily position the assembly into and around the joint space for cutting, severing, trimming and/or removing tissue to prepare the joint space for surgery. These and other features are discussed in greater detail herein.

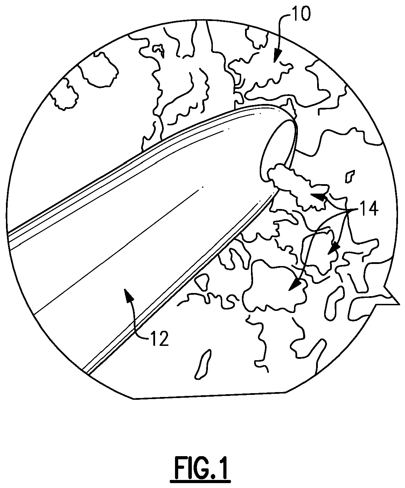

Figure 1 schematically illustrates a joint space 10 of a human or animal body that is being prepared for a surgical procedure, such as any arthroscopic or endoscopic procedure. The joint space 10 could be a knee joint, a shoulder joint, an ankle joint, a wrist joint or any other joint.

A surgical shaver assembly 12 may be positioned to access the joint space 10, such as through an arthroscopic or endoscopic portal, and may be moved into and around the joint space 10. In one embodiment, the surgical shaver assembly 12 is adapted for severing tissue 14 of the joint space 10. In this disclosure, the term "tissue" is intended to encompass both hard and soft body tissue. The surgical shaver assembly 12 may also remove debris to clean-up the joint space 10.

The surgical shaver assembly 12 prepares the joint space 10 for a surgical procedure. In one non-limiting embodiment, the surgical shaver assembly 12 is used to prepare a knee joint for surgery by trimming the meniscus and other tissue of the knee. In another embodiment, the surgical shaver assembly 12 is used to prepare a shoulder joint for surgery by trimming the rotator cuff and other tissue of the shoulder. Other surgical procedures are also contemplated as within the scope of this disclosure, and it should be understood that the surgical shaver assembly 12 of this disclosure can be used to trim any tissue.

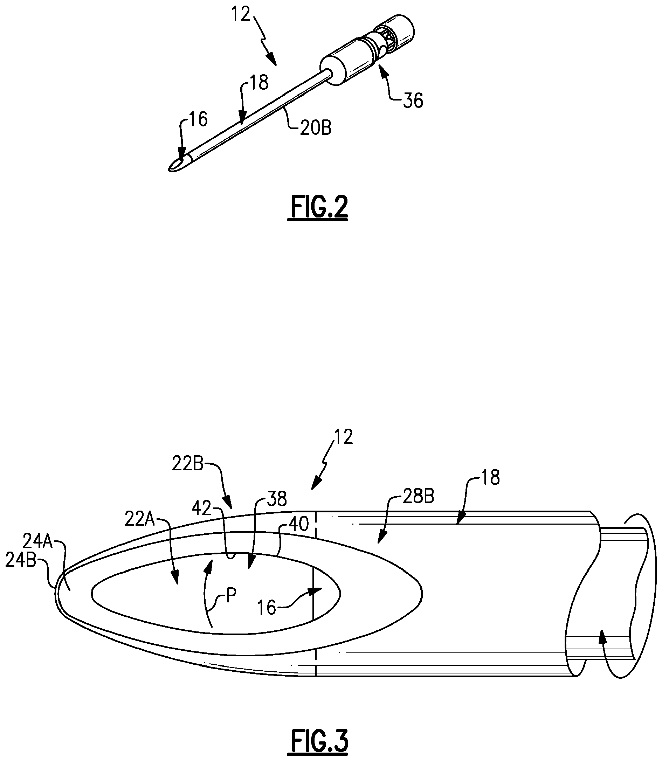

Figures 2-5 illustrate an exemplary surgical shaver assembly 12 that may be utilized to prepare a joint space, such as the joint space 10 of Figure 1, for surgery. The surgical shaver assembly 12 includes an inner tube 16 and an outer tube 18. The inner tube 16 is slidably and rotatably received by the outer tube 18, which generally surrounds the inner tube 16. When assembled, the inner tube 16 and the outer tube 18 include a concentric relationship relative to one another.

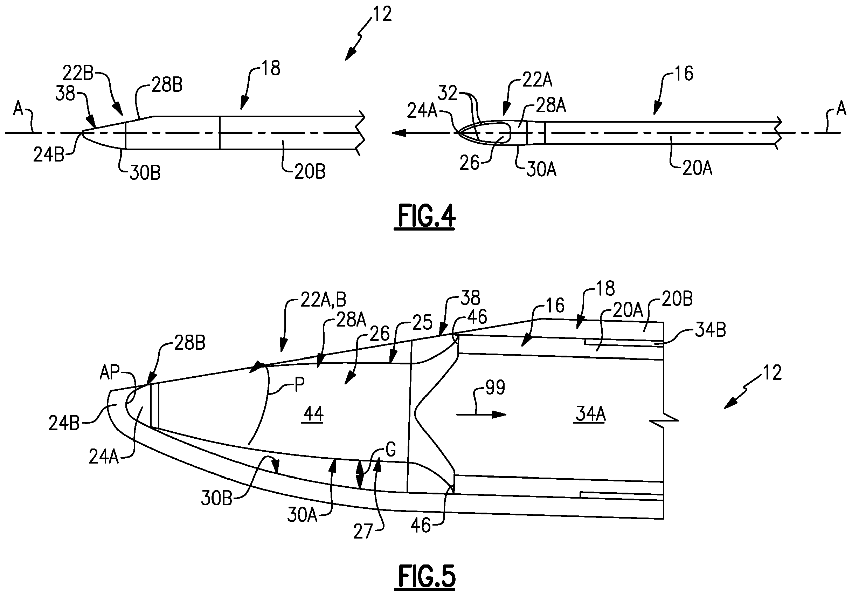

Both the inner tube 16 and the outer tube 18 include tubular bodies 20A, 20B that extend along a longitudinal axis A (see Figure 4). The tubular bodies 20A, 20B each define hollow passages 34A, 34B, respectively (see Figure 5). The hollow passage 34B of the outer tube 18 is sized to accommodate the inner tube 16 so that the inner tube 16 can slide and rotate relative to the outer tube 18. The hollow passage 34A of the inner tube 16 is sized so that tissue is easily removed through the inside of the surgical shaver assembly 12, as discussed in greater detail below.

The tubular bodies 20A, 20B additionally include distal portions 22A, 22B, respectively. The distal portions 22A, 22B may be conical shaped and may taper in a direction toward distal-most ends 24A, 24B of the distal portions 22A, 22B. In one embodiment, the distal portions 22A, 22B are not constantly tapered. In other words, discrete sections of the distal portions 22A, 22B may include a constant radius.

The distal portion 22A of the inner tube 16 includes a window 26 that extends entirely through the distal portion 22A to define a top opening 25 and a bottom opening 27 (see, for example, Figure 5). Stated another way, the window 26 is exposed or open at both a top surface 28A and a bottom surface 30A of the distal portion 22A of the inner tube 16. In one non-limiting embodiment, the window 26 is conical shaped (best illustrated in Figure 4). Cutting edges 32 circumscribe the periphery of the window 26 of the inner tube 16 at both the top opening 25 and the bottom opening 27 (see, for example, Figure 4).

The distal portion 22B of the outer tube 18 also includes a window 38. In one embodiment, the window 38 is oval shaped and includes a top opening 40 formed through a top surface 28B of the outer tube 18 (see, for example, Figure 3). A bottom surface 30B of the outer tube 18 is closed. In other words, unlike the window 26 of the inner tube 16, the window 38 of the outer tube 18 does not extend entirely through the distal portion 22B. A cutting edge 42 circumscribes the periphery of the window 38 of the outer tube 18 (see, for example, Figure 3).

During operation of the surgical shaver assembly 12, tissue may protrude into the window 38 of the outer tube 18 and the window 26 of the inner tube 16 when the windows 38, 26 align with one another. The portion of the tissue that is received within the windows 26, 38 is severed the cutting edges 32 as the inner tube 16 rotates within the outer tube 18 along a cutting edge path P (see Figure 3). The tissue that is sheared as the inner tube 16 rotates along the cutting edge path P can be aspirated and removed from the joint space through a conduit defined by the hollow passage 34A of the inner tube 16. The tissue may be aspirated and removed in the direction of arrow 99 shown in Figure 5.

The surgical shaver assembly 12 may additionally include a hub assembly 36 (see Figure 2) for connecting the surgical shaver assembly 12 to a hand-held surgical tool (not shown). The hand-held tool is configured to hold the surgical shaver assembly 12 and simultaneously rotate the inner tube 16 relative to the outer tube 18 to sever material of a joint space. In one embodiment, the hand-held surgical tool is a powered medical drill. Although not shown, the hand-held tool may additionally include a vacuum for applying suction pressure to the hollow passage 34A of the inner tube 16 to withdraw tissue or other debris that is severed during operation of the surgical shaver assembly 12.

Referring now to the cross-sectional view of Figure 5, the distal portion 22A of the inner tube 16 cooperates with the distal portion 22B of the outer tube 18 to define a cutting chamber 44. The cutting chamber 44 represents the area of the surgical shaver assembly 12 where tissue is severed by the sweeping motion of the inner tube 16 as it is moved along the cutting edge path P relative to the outer tube 18.

In one embodiment, a shoulder 46 is established within the cutting chamber 44 at a position between the inner tube 16 and the outer tube 18. In one embodiment, the shoulder 46 extends radially outward of the top opening 25 and bottom opening 27 of the window 26. In another embodiment, a gap G may extend between the bottom opening 27 of the window 26 of the inner tube 16 and the bottom surface 30B of the outer tube 18. The gap G may progressively reduce in size in a direction towards the distal-most ends 24A, 24B.

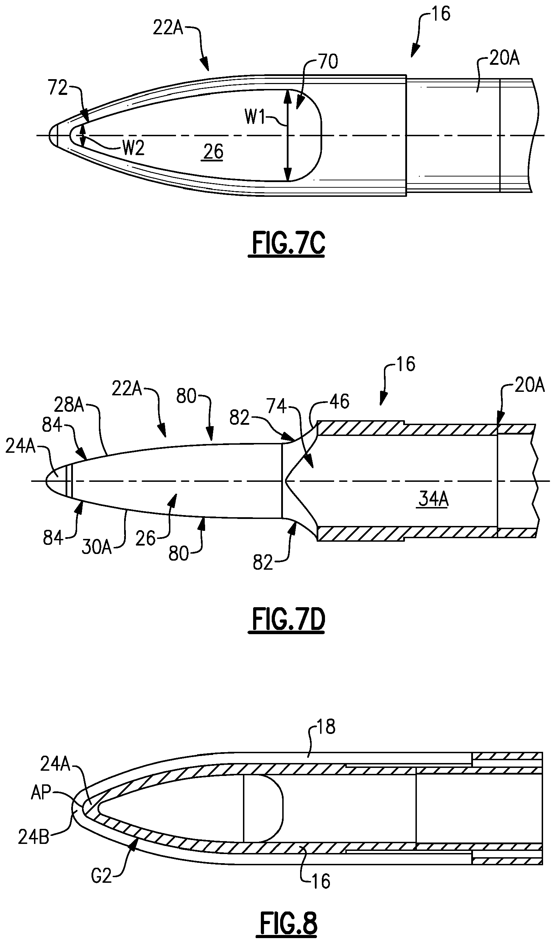

In a further non-limiting embodiment, due at least in part to the conical shapes of the distal portions 22A, 22B, the inner tube 16 contacts the outer tube 18 during rotation only at the distal-most end 24A. For example, an apex AP of the distal-most end 24A of the inner tube 16 may contact the distal-most end 24B of the outer tube 18. This self-centers the inner tube 16 relative to the outer tube 18 during rotation and enables a relatively tight gap G2 between the inner tube 16 and the outer tube 18 (see Figure 8). The tight tolerance established by the gap G2 induces a "scissor" effect when the inner tube 16 rotates within the outer tube 18 to render a highly effective cutting tool.

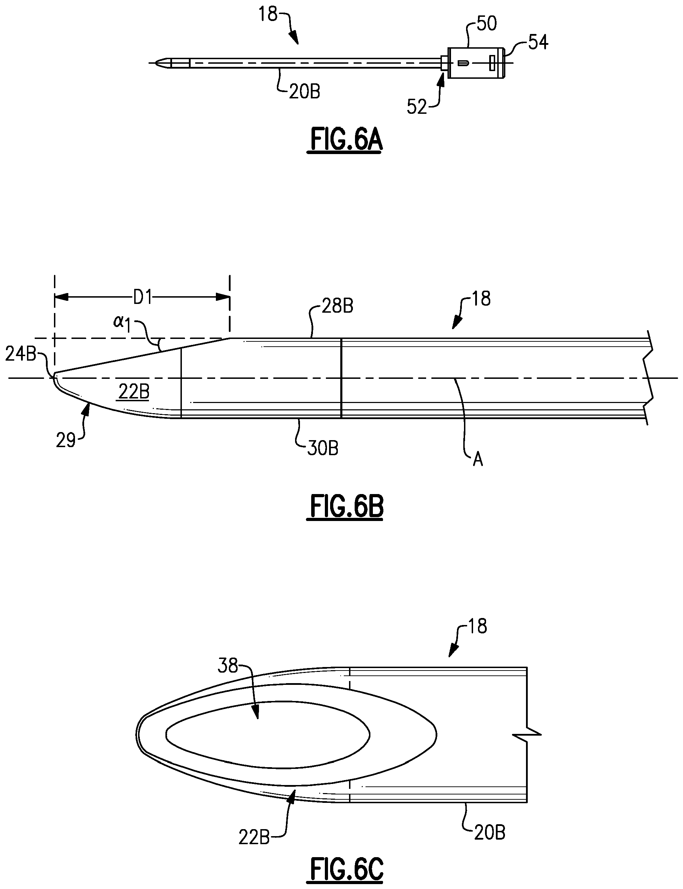

Figures 6A, 6B and 6C illustrate exemplary features of the outer tube 18 of the surgical shaver assembly 12 of Figures 1-5. Figure 6A illustrates an outer hub 50 that may be mounted to a proximal portion 52 of the tubular body 20B of the outer tube 18. The outer hub 50 may include a retaining ring 54. The outer hub 50 is suitable for mounting the surgical shaver assembly 12 to a hand-held surgical tool or to some other handpiece.

As shown in Figure 6B, the top surface 28B of the outer tube 18 may be tapered toward the distal-most end 24B at a taper angle α1 over a distance D1. The taper angle α1 defines the window 38 of the outer tube 18. The bottom surface 30B may include an arc 29 that rotates around the longitudinal axis A to configure the distal portion 22B in a "torpedo" shape such that the surgical shaver assembly 12 can be easily maneuvered into and around a joint space.

Referring to Figure 6C, the window 38 of the outer tube 18 may extend proximally of the distal portion 22B of the outer tube 18. In other words, the window 38 may extend at least partially into the tubular body 20B in a direction toward the proximal portion 52 (see Figure 6A).

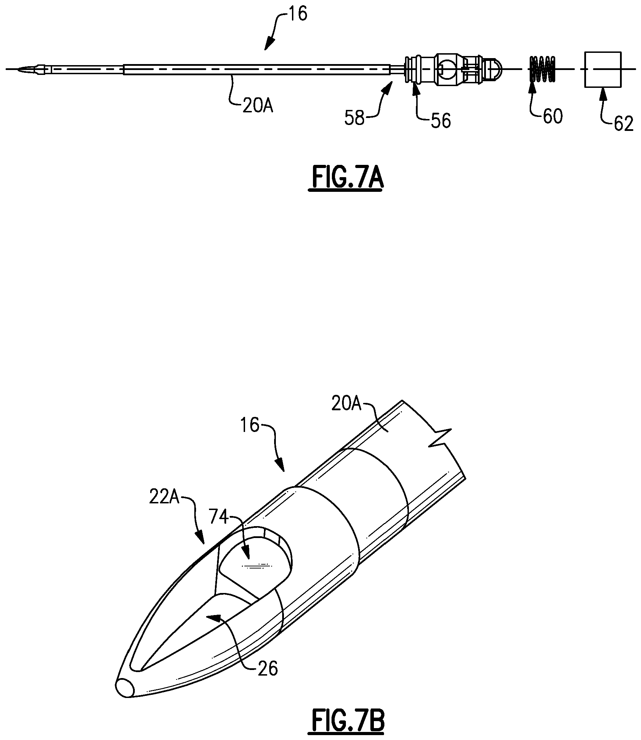

Figures 7A, 7B, 7C and 7D illustrate exemplary features of the inner tube 16 of the surgical shaver assembly 12 described above. As shown in Figure 7A, a hub 56 may be mounted to a proximal portion 58 of the tubular body 20A of the inner tube 16. In one non-limiting embodiment, the hub 56 is over-molded onto the proximal portion 58. The hub 56 may receive a spring 60 that is retained on the hub 56 via a spring retainer 62. The hub 56 is suitable for transmitting rotational motion provided by a motor drive of the hand-held surgical tool to the inner tube 16.

Referring to Figures 7B and 7C, the window 26 of the inner tube 16 may be conical shaped. The window 26 may include a first width W1 on a proximal side 70 and may include a second width W2 on a distal side 72 of the window 26 (best illustrated in Figure 7C). The first width W1 is greater than the second width W2, in this embodiment. In other words, the window 26 tapers from the proximal side 70 toward the distal side 72. The hollow passage 34A of the tubular body 20A of the inner tube 16 may include a distal opening 74 that opens into the window 26. In one embodiment, the distal opening 74 includes a V-shaped cross-section (see Figure 7D).

Referring primarily to Figure 7D, the distal portion 22A of the inner tube 16 may be contoured in a direction that extends from the tubular body 20A toward the distal-most end 24A of the distal portion 22A. For example, the top surface 28A and the bottom surface 30A of the distal portion 22A may include proximal sections 82 near the shoulder 46 that converge toward one another, intermediate sections 80 that diverge away from one another, and finally distal sections 84 that again converge toward one another toward the distal-most end 24A.

Although the different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should also be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims determine the true scope of the invention.

## Claims

1. A surgical shaver assembly (12), comprising:
an outer tube (18);
an inner tube (16) having a longitudinal axis, a proximal end and a distal end rotatably received within a hollow passage (34B) of said outer tube (18), said inner tube (16) including a distal portion (22A) and a window (26) that extends through said distal portion (22A);
wherein said inner tube (16) is self-centering relative to said outer tube (18) to establish a tight gap between said inner tube (16) and said outer tube (18), whereby said tight gap induces a scissor effect when said inner tube (16) rotates within said outer tube (18) to sever tissue;
whereby the inner tube (16) is self-centering via an apex (AP) at a distal end of the inner tube (16) received within a conical shaped distal portion (22B) of the outer tube (18); and
wherein, in a direction from the proximal end to the distal end of the inner tube (16), said distal portion (22A) of said inner tube (16) includes a top surface (28A) and a bottom surface (30A) that include proximal sections (82), wherein the top and bottom surfaces (28A, 30A) converge toward the longitudinal axis, intermediate sections (80), wherein the top and bottom surfaces (28A, 30A) diverge away from the longitudinal axis, and distal sections (84), wherein the top and bottom surfaces (28A, 30A) converge toward the longitudinal axis, wherein the intermediate sections (80) are between the proximal sections (82) and the distal sections (84).

2. The assembly as recited in claim 1, wherein said inner tube (16) includes a tubular body (20A) that includes said distal portion (22A), said distal portion (22A) tapering in direction that extends from said tubular body (20A) toward a distal-most end (24A) of said inner tube (16).

3. The assembly as recited in claim 1 or 2, wherein the top surface (28A) includes a top opening (25) and the bottom surface (30A) includes a bottom opening (27), said window (26) extending from said top opening (25) to said bottom opening (27).

4. The assembly as recited in claim 3, wherein cutting edges (32) circumscribe the periphery of both said top opening (25) and said bottom opening (27).

5. The assembly as recited in any preceding claim, wherein said outer tube (18) includes a top surface (28B) that includes a top opening and a bottom surface (30B) that is closed, said top opening establishing a window (38) of said outer tube (16).

6. The assembly as recited in claim 5, wherein said inner tube (16) is configured to rotate within said outer tube (18) to sever tissue that extends through said window (38) of said outer tube (18) and said window (26) of said inner tube (16) when said windows become aligned.

7. The assembly as recited in any preceding claim, wherein said inner tube (16) includes a bottom opening (27) and said outer tube (18) includes a bottom surface (30B), and comprising a gap (G) that extends between said bottom opening (27) and said bottom surface (30B).

8. The assembly as recited in any preceding claim, wherein a top surface (28B) of said outer tube (18) tapers at a first taper angle toward a distal-most end of said outer tube (18) to establish a window (38) of said outer tube (18).

9. The assembly as recited in any preceding claim, wherein a bottom surface (30B) of said outer tube (18) includes an arc (29) that rotates around a longitudinal axis of said outer tube (18) to configure a distal portion of said outer tube (18) in a "torpedo" shape.

10. The assembly as recited in any preceding claim, wherein said outer tube (18) includes a window (38) that extends into a portion of a tubular body (20B) of said outer tube (18) that is proximal to a distal portion of said outer tube (18).

11. The assembly as recited in any preceding claim, wherein said window (26) of said inner tube (16) includes a first width (W1) at a proximal side (70) and a second width (W2) at a distal side (72), said first width larger than said second width.

12. The assembly as recited in any preceding claim, wherein said inner tube (16) includes a tubular body (20A) that establishes a hollow passage (34A), and a distal opening (74) of said hollow passage (34A) opens into said window (26) of said inner tube (16).

13. The assembly as recited in claim 11, wherein said distal opening (74) includes a V-shaped cross-section.

## Patentansprüche

1. Chirurgische Rasiereranordnung (12), umfassend:
eine Außenröhre (18);
eine Innenröhre (16) mit einer Längsachse, einem proximalen Ende und einem distalen Ende, die drehbar in einem hohlen Durchgang (34B) der Außenröhre (18) aufgenommen sind, wobei die Innenröhre (16) einen distalen Abschnitt (22A) und ein Fenster (26) beinhaltet, das sich durch den distalen Abschnitt (22A) erstreckt;
wobei sich die Innenröhre (16) in Bezug auf die Außenröhre (18) selbst zentriert, um einen engen Spalt zwischen der Innenröhre (16) und der Außenröhre (18) herzustellen, wodurch der enge Spalt einen Schereneffekt hervorruft, wenn sich die Innenröhre (16) innerhalb der Außenröhre (18) dreht, um das Gewebe abzutrennen;
wobei die Innenröhre (16) über einen Scheitelpunkt (AP) an einem distalen Ende der Innenröhre (16), die in einem konisch geformten distalen Abschnitt (22B) der Außenröhre (18) aufgenommen ist, selbstzentrierend ist; und
wobei in einer Richtung vom proximalen Ende zum distalen Ende der Innenröhre (16), der distale Abschnitt (22A) der Innenröhre (16) eine obere Oberfläche (28A) und eine untere Oberfläche (30A) aufweist, die proximale Abschnitte (82) aufweisen, wobei die obere und untere Oberfläche (28A, 30A) in Richtung der Längsachse konvergieren, und Zwischenabschnitte (80), wobei die obere und untere Oberfläche (28A), 30A) von der Längsachse weg divergieren, und distale Abschnitte (84), wobei die oberen und unteren Oberflächen (28A, 30A) in Richtung der Längsachse konvergieren, wobei die Zwischenabschnitte (80) zwischen den proximalen Abschnitten (82) und den distalen Abschnitten (84) angeordnet sind.

2. Anordnung nach Anspruch 1, wobei die Innenröhre (16) einen rohrförmigen Körper (20A) aufweist, der den distalen Abschnitt (22A) aufweist, wobei sich der distale Abschnitt (22A) in der Richtung verjüngt, die sich von dem rohrförmigen Körper (20A) aus erstreckt zu einem am weitesten distal gelegenen Ende (24A) der Innenröhre (16).

3. Baugruppe nach Anspruch 1 oder 2, wobei die obere Fläche (28A) eine obere Öffnung (25) und die untere Fläche (30A) eine untere Öffnung (27) aufweist, wobei sich das Fenster (26) von der oberen Öffnung (25) zu der unteren Öffnung (27) erstreckt.

4. Baugruppe nach Anspruch 3, wobei Schneidkanten (32) den Umfang sowohl der oberen Öffnung (25) als auch der unteren Öffnung (27) umgeben.

5. Baugruppe nach einem der vorhergehenden Ansprüche, wobei die Außenröhre (18) eine obere Fläche (28B) mit einer oberen Öffnung und eine geschlossene untere Fläche (30B) aufweist, wobei die obere Öffnung ein Fenster (38) der Außenröhre(16) bildet.

6. Anordnung nach Anspruch 5, wobei die Innenröhre (16) so konfiguriert ist, dass sie sich innerhalb der Außenröhre (18) dreht, um Gewebe abzutrennen, das sich durch das Fenster (38) der Außenröhre (18) und das Fenster (26) der Innenröhre (16) erstreckt, wenn die Fenster ausgerichtet werden.

7. Anordnung nach einem der vorhergehenden Ansprüche, wobei die Innenröhre (16) eine untere Öffnung (27) und die Außenröhre (18) eine untere Fläche (30B) aufweist und einen Spalt (G) aufweist, der sich zwischen der unteren Öffnung (27) und der unteren Fläche (30B) erstreckt.

8. Baugruppe nach einem der vorhergehenden Ansprüche, wobei sich eine obere Oberfläche (28B) der Außenröhre (18) in einem ersten Verjüngungswinkel zu einem am weitesten distal gelegenen Ende der Außenröhre (18) hin verjüngt, um ein Fenster (38) der Außenröhre (18) herzustellen.

9. Baugruppe nach einem der vorhergehenden Ansprüche, wobei eine untere Fläche (30B) der Außenröhre (18) einen Bogen (29) aufweist, der sich um eine Längsachse der Außenröhre (18) dreht, um einen distalen Abschnitt der Außenröhre (18) in einer "Torpedo"-Form zu bilden.

10. Anordnung nach einem der vorhergehenden Ansprüche, wobei die Außenröhre (18) ein Fenster (38) aufweist, das sich in einen Abschnitt eines rohrförmigen Körpers (20B) der Außenröhre (18) erstreckt, der sich in der Nähe eines distalen Abschnitts der Außenröhre (18) befindet.

11. Baugruppe nach einem der vorhergehenden Ansprüche, wobei das Fenster (26) der Innenröhre (16) eine erste Breite (W1) an einer proximalen Seite (70) und eine zweite Breite (W2) an einer distalen Seite (72) beinhaltet, wobei die erste Breite größer als die zweite Breite ist.

12. Baugruppe nach einem der vorhergehenden Ansprüche, wobei die Innenröhre (16) einen rohrförmigen Körper (20A) beinhaltet, der einen Hohlkanal (34A) bildet, und sich eine distale Öffnung (74) des Hohlkanals (34A) in das Fenster (26) der Innenröhre (16) öffnet.

13. Baugruppe nach Anspruch 11, wobei die distale Öffnung (74) einen V-förmigen Querschnitt aufweist.

## Revendications

1. Ensemble de rasage chirurgical (12), comprenant :
un tube externe (18) ;
un tube interne (16) ayant un axe longitudinal, une extrémité proximale et une extrémité distale reçue avec faculté de rotation dans un passage creux (34B) dudit tube externe (18), ledit tube interne (16) comprenant une partie distale (22A) et une fenêtre (26) qui s'étend à travers ladite partie distale (22A) ;
dans lequel ledit tube interne (16) est autocentré par rapport audit tube externe (18) pour établir un espace étroit entre ledit tube interne (16) et ledit tube externe (18), moyennant quoi ledit espace étroit induit un effet de cisaillement lorsque ledit tube interne (16) tourne dans ledit tube externe (18) pour couper du tissu ;
moyennant quoi le tube interne (16) est autocentré via un sommet (AP) au niveau d'une extrémité distale du tube interne (16) reçue dans une partie distale de forme conique (22B) du tube externe (18) ; et
dans lequel, dans une direction de l'extrémité proximale à l'extrémité distale du tube interne (16), ladite partie distale (22A) dudit tube interne (16) comprend une surface supérieure (28A) et une surface inférieure (30A) qui comprennent des sections proximales (82), dans lequel les surfaces supérieure et inférieure (28A, 30A) convergent vers l'axe longitudinal, des sections intermédiaires (80), dans lequel les surfaces supérieure et inférieure (28A, 30A) divergent depuis l'axe longitudinal, et des sections distales (84), dans lequel les surfaces supérieure et inférieure (28A, 30A) convergent vers l'axe longitudinal, dans lequel les sections intermédiaires (80) se trouvent entre les sections proximales (82) et les sections distales (84).

2. Ensemble selon la revendication 1, dans lequel ledit tube interne (16) comprend un corps tubulaire (20A) qui comprend ladite partie distale (22A), ladite partie distale (22A) s'effilant dans une direction qui s'étend dudit corps tubulaire (20A) vers une extrémité la plus distale (24A) dudit tube interne (16).

3. Ensemble selon la revendication 1 ou 2, dans lequel la surface supérieure (28A) comprend une ouverture supérieure (25) et la surface inférieure (30A) comprend une ouverture inférieure (27), ladite fenêtre (26) s'étendant de ladite ouverture supérieure (25) à ladite ouverture inférieure (27) .

4. Ensemble selon la revendication 3, dans lequel des bords de coupe (32) circonscrivent la périphérie à la fois de ladite ouverture supérieure (25) et de ladite ouverture inférieure (27).

5. Ensemble selon l'une quelconque des revendications précédentes, dans lequel ledit tube externe (18) comprend une surface supérieure (28B) qui comprend une ouverture supérieure et une surface inférieure (30B) qui est fermée, ladite ouverture supérieure établissant une fenêtre (38) dudit tube externe (16).

6. Ensemble selon la revendication 5, dans lequel ledit tube interne (16) est conçu pour tourner dans ledit tube externe (18) pour couper du tissu qui s'étend à travers ladite fenêtre (38) dudit tube externe (18) et ladite fenêtre (26) dudit tube interne (16) lorsque les fenêtres deviennent alignées.

7. Ensemble selon l'une quelconque des revendications précédentes, dans lequel ledit tube interne (16) comprend une ouverture inférieure (27) et ledit tube externe (18) comprend une surface inférieure (30B), et comprenant un espace (G) qui s'étend entre ladite ouverture inférieure (27) et ladite surface inférieure (30B).

8. Ensemble selon l'une quelconque des revendications précédentes, dans lequel une surface supérieure (28B) dudit tube externe (18) s'effile selon un premier angle incliné vers une extrémité la plus distale dudit tube externe (18) pour établir une fenêtre (38) dudit tube externe (18).

9. Ensemble selon l'une quelconque des revendications précédentes, dans lequel une surface inférieure (30B) dudit tube externe (18) comprend un arc (29) qui tourne autour d'un axe longitudinal dudit tube externe (18) pour configurer une partie distale dudit tube externe (18) en forme de « torpille ».

10. Ensemble selon l'une quelconque des revendications précédentes, dans lequel ledit tube externe (18) comprend une fenêtre (38) qui s'étend dans une partie du corps tubulaire (20B) dudit tube externe (18) qui est proche d'une partie distale dudit tube externe (18).

11. Ensemble selon l'une quelconque des revendications précédentes, dans lequel ladite fenêtre (26) dudit tube interne (16) comprend une première largeur (W1) au niveau d'un côté proximal (70) et une seconde largeur (W2) au niveau d'un côté distal (72), ladite première largeur étant plus grande que ladite seconde largeur.

12. Ensemble selon l'une quelconque des revendications précédentes, dans lequel ledit tube interne (16) comprend un corps tubulaire (20A) qui établit un passage creux (34A), et une ouverture distale (74) dudit passage creux (34A) s'ouvre dans ladite fenêtre (26) dudit tube interne (16) .

13. Ensemble selon la revendication 11, dans lequel ladite ouverture distale (74) comprend une section transversale en V.
